# EUROPEAN PATENT SPECIFICATION

(11) **EP 1 742 645 B1**
(45) Date of publication and mention of the grant of the patent: **26.01.2011**
(21) Application number: 05735983.8
(22) Date of filing: 25.04.2005
(51) Int. Cl.: A61K 31/765, A61P 1/10, A61K 9/20, A61K 33/14

(54) **COMPRESSED PHARMACEUTICAL COMPOSITIONS COMPRISING PEG AND ELECTROLYTES**
KOMPRIMIERTE PHARMAZEUTISCHE ZUSAMMENSETZUNGEN MIT PEG UND ELEKTROLYTEN
COMPOSITIONS PHARMACEUTIQUES COMPRIMEES COMPRENANT PEG ET DES ELECTROLYTES

(30) Priority: 23.04.2004 GB 0409104
(43) Date of publication of application: 17.01.2007
(60) Divisional application: 10177007.1
(73) Proprietor: Norgine BV, 1101 CA Amsterdam Zuid-Oost (NL)
(72) Inventor: Barras, Norman, Harefield, Uxbridge, Middlesex UB9 6NS (GB); Cox, Ian, Hengoed, Mid Glamorgan CF82 8SJ (GB)
(74) Representative: Brady, Paul Andrew
(86) International application number: PCT/GB2005/001555
(87) International publication number: WO 2005/102364

(56) References cited:
- WO-A-87/00754
- WO-A-2005/049049
- WO-A-2005/051361
- US-A- 3 121 663
- GONZALEZ-RODRIGUEZ M.L. ET AL: "In vitro release of sodium diclofenac from a central core matrix tablet aimed for colonic drug delivery." EUROPEAN JOURNAL OF PHARMACEUTICAL SCIENCES, 20/1 (125-131). REFS: 24 ISSN: 0928-0987 CODEN: EPSCED, 2003, XP002303014
- GONZALEZ-RODRIGUEZ M L ET AL: "Design and evaluation of a new central core matrix tablet" INTERNATIONAL JOURNAL OF PHARMACEUTICS 1997 NETHERLANDS, vol. 146, no. 2, 1997, pages 175-180, XP002303015 ISSN: 0378-5173

## Description

The present invention relates to compositions for the treatment of constipation, faecal impaction, faecal retention, intestinal gas and cramping, flatulence, and also to compositions for orthostatic lavage, colon evacuation or colon cleansing. The invention relates in particular to compositions for the treatment of constipation. The invention further relates to methods of use of such compositions.

### General Background

Constipation is a widespread condition which generally gives rise to discomfort. The physical presence of faeces retained in the colon and/or the rectum gives rise to a feeling of malaise and headaches. In extreme cases of prolonged constipation dyschezia may result from the presence of scybala or faecaliths in the rectum.

Numerous treatments of constipation have been developed, including dietary manipulation (e.g. increasing the fibre content of the diet and removing foods considered to be constipation causing), laxatives and enemas. Laxatives are agents that promote and assist defecation. Osmotic laxatives act to retain water in the colonic lumen thereby counteracting the normal dehydrating action of the colon. By suppressing the dehydration action of the colon, the osmotic laxative produces a faecal stream which is softer, bulkier and easier to expel.

A number of osmotic laxative treatments currently in use comprise polyethylene glycol (PEG). Such compositions may also include electrolytes. One laxative that comprises PEG and electrolytes that is currently on the market is Movicol®. Movicol is available in the UK and other countries from Norgine Limited (Chaplin House, Widewater Place, Moorhall Road, Harefield, Middlesex UB9 6NS, UK). It is supplied in sachets, each containing 13.8g of powder. Each sachet contains the following ingredients: Macrogol (Polyethylene Glycol) 3350: 13.125g, Sodium Chloride: 350.7mg; Sodium Bicarbonate: 178.5mg; Potassium Chloride: 46.6mg; and flavouring and sweetener (trace amounts). The sachets include instructions for making the powder up in 125mL of water.

Many patients who take Movicol or other PEG-based laxatives in powder, granular, solution, or in suspension form find the taste unpleasant and very salty. Those negative sensations lead to patient compliance being adversely affected.

Colon cleansing is important prior to numerous diagnostic and surgical procedures, for example before colonoscopy, barium enema examination or colon surgery. It is also useful for preventing infection after surgery on the lower intestine. Colon cleansing is also known as colon clearing.

One method of colon cleansing is orthostatic intestinal lavage, in which a large volume of an electrolyte solution is ingested, either by drinking or by infusion through a nasogastric tube. Such lavage solutions are also known as bowel or gut lavage solutions. Consumption of the solution results in volume-induced diarrhoea and thus cleansing of the colon. Most commonly used lavage solutions include PEG. In 1980, Davis and co-workers reported the development of a lavage solution, that they described as being associated with minimal water and electrolyte absorption or secretion (Davis G.R. et al., Gastroenterology, 1980, 78, 991-995). The solution included sodium sulphate and polyethylene glycol. In addition to sodium sulphate (40.0 mM, 5.68g/L) and polyethylene glycol (PEG 4000 "carbowax", 64g/L), the solution described by Davis *et al.* comprises sodium chloride (25mM, 1.463g/L), potassium chloride (10mM, 0.745g/L), sodium bicarbonate (20mM, 1.680g/L) and water. The solution was administered in a quantity of 4 litres and it was shown to be effective in cleansing the gastrointestinal tract. A related solution has been commercialised under the trade name GoLYTELY® (Braintree Laboratories Inc, Braintree, Massachusetts, U.S.A.). WO 87/00754 discloses low-sodium laxative and lavage formulation solutions comprising specified proportions of polyethylene glycol, sodium ions, potassium ions, chloride, ions and bicarbonate ions.

The commercially available GoLYTELY® composition as available after August 1996 and at the time of filing, is supplied in dry powder form comprising sodium sulphate (40.0 mM, 5.685g/L), sodium chloride (25mM, 1.464g/L), potassium chloride (10mM, 0.743g/L), sodium bicarbonate (20mM, 1.685g/L) and PEG 3350 polyethylene glycol (59g/L) for making up to 4 litres. GoLYTELY® is also supplied in aqueous solution.

As with laxative compositions, many patients who take GoLYTELY® or other PEG-based cleansing solutions find the taste of the solutions unpleasant. Those negative sensations lead to patient compliance being adversely affected.

Faecal impaction, also known as faecal retention is the formation of a firm impassable mass of stool in the rectum or distal colon. Faecal impaction is often treated using methods similar to those described above for constipation and colon cleansing. A larger dose is generally needed than in the case of constipation treatment. Accordingly, the recommended treatment using Movicol comprises taking 8 sachets of Movicol (each containing the quantities of ingredients mentioned above) daily dissolved in 1 litre water and drunk within 6 hours, usually for a maximum of 3 days.

As with laxative and cleansing compositions, many patients who take Movicol® or other PEG-based compositions for faecal impaction find the taste of the solutions unpleasant. Those negative sensations lead to patient compliance being adversely affected.

US patent 3,121,663 discloses a suppository composition consisting essentially of polyethylene glycol which is solid at normal temperatures, sodium bicarbonate and sodium biphosphate.

There remains a requirement for laxatives, faecal impaction treatments and lavage preparations with a more pleasant taste (less salty) and a more pleasant mouthfeel.

Gonzalez-Rodriguez M.L. et al, European Journal of Pharmaceutical Sciences 20 (2003) 125-131 discloses a sodium diclofenac formulation for colonic drug delivery in which the central core is formed by a solid dispersion of the drug into polyethylene glycol 4000. The design of the core matrix formulation is illustrated in Gonzalez-Rodriguez M.L. et al, International Journal of Pharmaceutics 146 (1997).

### Description of the invention

The invention provides a compressed pharmaceutical composition comprising
- polyethylene glycol of molecular weight from 2000 to 4500, wherein the polyethylene glycol makes up from 80 to 99.5% by weight of the composition,
- one or more electrolytes
for the treatment of constipation, faecal impaction, faecal retention, intestinal gas and cramping, or flatulence; or for orthostatic lavage, colon evacuation or colon cleansing. In particular, there is provided such a composition for use in the treatment of constipation, faecal impaction, faecal retention, intestinal gas and cramping, flatulence. There is also provided a composition of the invention for orthostatic lavage, colon evacuation or colon cleansing.

It has been found, surprisingly, that a compressed pharmaceutical composition comprising PEG, for example a solid tablet, is significantly more palatable than previously proposed compositions and is effective when administered. The compressed pharmaceutical composition of the invention comprising polyethylene glycol has been found to have a more agreeable taste and a more agreeable mouthfeel than previously proposed liquid or dry powder or granular compositions.

In the compressed pharmaceutical compositions of the invention polyethylene glycol makes up from 80 to 99.5% by weight of the composition. The remainder of the composition is made up of electrolytes and optionally one or more components selected from excipients, sweeteners and flavouring agents.

The polyethylene glycol (PEG) used in a composition of the present invention preferably has an average molecular weight of 2000 or greater. Preferably the PEG has an average molecular weight of 2500 or greater. Preferably the PEG has an average molecular weight of 4500 or lower. For example the PEG may be PEG 3350 or PEG 4000. PEG 3350 is most preferred. That product is available commercially, for example, from Dow Chemical Co., Clariant GmbH or BASF under the tradename Macrogol 3350.

The PEG is present in the pharmaceutical preparations of the invention in an amount and in a concentration necessary to achieve pharmaceutical efficacy in the patient. In the currently marketed PEG-containing product Movicol® for adult use, each sachet contains 13.125g of PEG 3350 and adults are instructed to take from 1 to 3 sachets per day (i.e. from 13.125g to 39.375g PEG per day) for the treatment of constipation. The same total quantities of PEG are also appropriate in the case of the compressed pharmaceutical preparations of the present invention for the treatment of constipation. Accordingly, a composition of the invention may comprise more than 5g of PEG, more preferably more than 10g of PEG. A composition of the invention may comprise less than 20g of PEG, more preferably less than 15g of PEG. A composition of the invention may comprise from 5 to 20g of PEG, preferably from 10 to 15g of PEG, for example 13.125g of PEG. A given composition may optionally be presented in a divided dose form such that a number of smaller units are ingested and the total PEG taken is approximately from 10 to 15g.

A particularly convenient compressed composition is a composition comprising from 1.5 to 5.5g of PEG, preferably from 2.0 to 3.3g of PEG, for example 2.5g or 2.625g of PEG. The patient would be advised to take from 3 to 5 such tablets to chew or suck to make up the 10 to 15g of PEG for a full dose.

Alternatively, two, three or four dosage units may be appropriate, such that the two, three or four dosage units are taken at the same time to make up the effective dose. Thus, a dosage unit composition presenting half of a dose may comprise from 2 to 10g of PEG, preferably from 5 to 7.5g of PEG, for example 6.563g of PEG. Patients are advised to take from 2 to 6 such unit doses of composition per day, such that they receive from 13.125g to 39.375g PEG per day. Similarly, a dosage unit composition presenting a third of a dose may comprise from 1.67 to 6.67g of PEG, preferably from 3.33 to 5g of PEG, for example 4.375g of PEG. Patients are advised to take from 3 to 9 such unit doses of composition per day, such that they receive from 13.125g to 39.375g PEG per day. Similarly, a dosage unit composition presenting a quarter of a dose may comprise from 1 to 5g of PEG, preferably from 2.5 to 3.75g of PEG, for example 3.281 g of PEG. Patients are advised to take from 4 to 12 such unit doses of composition per day, such that they receive from 13.125g to 39.375g PEG per day.

For the treatment of faecal impaction, also known as faecal retention, adults are instructed to take from 6 to 10 sachets of Movicol® (appropriately dissolved in water) per day (i.e. from 78.75g to 131.25g PEG per day). The number of divided doses of the composition of the invention that are appropriate are analogous to those known in the art that provide similar quantities of PEG, i.e. for example from 15 to 65 doses of a composition of the invention comprising from 2 to 5g, for example 4g, of PEG may be administered, such that the patient receives from 78.75g to 131.25g PEG per day.

Paediatric products currently on the market comprise smaller quantities of PEG per sachet than their adult counterparts. For example, Movicol® for the treatment of faecal impaction in children (sold under the tradename Movicol Paediatric Plain) contains 6.563g of PEG 3350 with instructions for making up to 62.5mL of solution in water. For paediatric use, the adult half dose form mentioned above may be suitable as a full dose. Thus, a composition of the invention for paediatric use may comprise from 2 to 10g of PEG, more preferably from 5 to 7.5g of PEG, for example 6.563g of PEG. Paediatric patients with faecal impaction are advised to take from 2 to 12 such units of composition per day, depending on their age. It is common to treat paediatric faecal impaction by taking a course of Movicol Paediatric Plain over 7 days with increasing doses as the week progresses. A 2-4 year old may be advised to take 2 doses of the composition of the invention on the first day increasing to 8 doses on the 7th day, whilst a 5 to 11 year old may be advised to take 4 doses on the first day increasing to 12 doses on the 7^{th} day.

For colon cleansing, the currently marketed compositions mentioned in the introduction above are administered in quantities such that the patient receives in the region of 200 to 300g of PEG in one dose. Similar quantities of PEG are also appropriate in the case of the compressed pharmaceutical preparations of the current invention for colon cleansing. Accordingly, a composition of the invention may comprise from 200 to 300g of PEG. A given composition may optionally be presented in a divided dose form, such that two, three or more presented doses are taken at the same time to make up the effective dose. Such divided dose forms may provide an appropriate fraction of the total dose. In the case where the composition of the invention is provided in a form comprising from 5 to 7.5g of PEG, for example 6.563g of PEG, the patient would be recommended to take from 25 to 60 of the composition units. It may be preferable to dissolve the compressed pharmaceutical composition in water before taking the composition.

A pharmaceutical composition of the present invention comprises one or more electrolytes. Polyethylene glycol is considered to be effective as a laxative by virtue of its osmotic action in the gut. Polyethylene glycol is not absorbed by the gut to any significant exigent. When taken on its own, polyethylene glycol has an electrolyte leaching effect, and so it can lead to a drop in electrolyte levels in the patient. Accordingly, it is preferred for the compositions of the invention to comprise one or more electrolytes. Sodium ions and potassium ions are particularly suitable. Those ions may be present in the form of any suitable salts, for example as their chloride, bicarbonate, sorbate, benzoate, acetate, carbonate, citrate, fumarate, gluconate, malate, nitrate, phosphate, succinate, sulphate or tartrate salts. Chloride and bicarbonate salts are particularly preferred, for example sodium chloride, potassium chloride and sodium bicarbonate.

Preferably the electrolytes are selected from sodium chloride, sodium bicarbonate or potassium chloride. Most preferably a composition of the invention comprises all of these electrolytes.

In a preferred formulation comprising 13.125g of polyethylene glycol in one dose, there is preferably present sodium chloride in an amount of from 0.1 to 1.0g, more preferably from 0.2 to 0.6g, for example from 0.3 to 0.5g, for example 0.3507g. For compositions comprising different amounts of polyethylene glycol, quantities of sodium chloride are altered in proportion to the amount of polyethylene glycol present.

In a preferred formulation comprising 2.5g of polyethylene glycol in one dose, there is preferably present sodium chloride in an amount of from 0.01 to 0.2g, more preferably from 0.03 to 0.11g, for example from 0.05 to 0.09g, for example 0.0668g.

In a preferred formulation comprising 13.125g of polyethylene glycol in one dose, there is preferably present sodium bicarbonate in an amount of from 0.05 to 0.5g, more preferably from 0.1 to 0.3g, for example from 0.15 to 0.25g, for example 0.1785g. For compositions comprising different amounts of polyethylene glycol, quantities of sodium bicarbonate are altered in proportion to the amount of polyethylene glycol present.

In a preferred formulation comprising 2.5g of polyethylene glycol in one dose, there is preferably present sodium bicarbonate in an amount of from 0.01 to 0.1g, more preferably from 0.02 to 0.06g, for example from 0.03 to 0.05g, for example 0.0340g.

In a preferred formulation comprising 13.125g of polyethylene glycol in one dose, there is preferably present potassium chloride in an amount of from 0.01 to 0.15g, more preferably from 0.02 to 0.1g, for example from 0.03 to 0.06g, for example 0.0466g. For compositions comprising different amounts of polyethylene glycol, quantities of potassium chloride are altered in proportion to the amount of polyethylene glycol present.

In a preferred formulation comprising 2.5g of polyethylene glycol in one dose, there is preferably present potassium chloride in an amount of from 0.002 to 0.03g, more preferably from 0.004 to 0.019g, for example from 0.008 to 0.01g, for example 0.00888g.

The compressed pharmaceutical compositions of the invention may include sodium sulphate.

In a preferred formulation comprising 13.125g of polyethylene glycol in one dose, there may be present sodium sulphate in an amount of from 0.2 to 2g, more preferably from 0.5 to 1.6g, for example from 0.7 to 1.3g, for example 0.9375g.

In a preferred formulation comprising 2.5g of polyethylene glycol in one dose, there is preferably present sodium sulphate in an amount of from 0.04 to 0.5g, more preferably from 0.1 to 0.3g, for example from 0.15 to 0.25g, for example 0.179g

The compressed pharmaceutical compositions of the invention may comprise from 5 to 20g of PEG with a molecular weight of 2500 to 4500, 0.1 to 1.0g of sodium chloride, 0.05 to 0.5g of sodium bicarbonate, 0.01 to 0.15g of potassium chloride, and optionally 0.2 to 2g of sodium sulphate.

The compressed pharmaceutical compositions of the invention may comprise from 1.5 to 5.5g of PEG with a molecular weight of 2500 to 4500, 0.0401 to 0.147g of sodium chloride, 0.0204 to 0.0748g of sodium bicarbonate, 0.00533 to 0.0195g of potassium chloride, and optionally 0.107 to 0.393g of sodium sulphate.

The compressed pharmaceutical compositions of the invention may comprise ascorbic acid or an ascorbate salt.

The compressed pharmaceutical compositions of the invention may include one or more pharmaceutically acceptable excipients, in particular compression excipients. Suitable excipients include magnesium stearate, cellulose and derivatives of cellulose, starch and derivatives of starch, lactose and derivatives of lactose, dextrates, mannitols and sorbitols. Particularly in the case of an excipient selected from dextrates, mannitols and sorbitols, the compositions may have been spray dried during their manufacture.

The compressed pharmaceutical compositions of the invention preferably comprise no other active components apart from PEG. In one embodiment the compressed pharmaceutical compositions of the invention comprise no other components with laxative activity.

The compressed pharmaceutical compositions of the invention preferably comprise one or more sweeteners. Artificial or naturally derived sweeteners may be used alone or in admixture. Suitable sweeteners include, but are not limited to, saccharin, sodium saccharin, sodium cyclamate, acesulfame potassium, thaumatin, neohesperidin dihydrochalcone, ammonium glycyrrhizinate and aspartame. The sweetener may make up between from about 0.01 to 1.0% by weight of the final composition, more preferably about 0.025 to 0.25% by weight, for example from 0.07 to 0.08% by weight. Where two sweeteners are used in admixture, the ratios between the two sweeteners may be in the range of about 1:10 to 10:1 by weight.

The compressed compositions of the invention preferably comprise one or more flavouring agents. Suitable flavourings are available from International Flavors & Fragrances Inc. (IFF (GB) Ltd, Duddery Hill, Haverhill, Suffolk, CB9 8LG, United Kingdom) and include flavourings commonly used in foodstuffs and pharmaceuticals. Examples of suitable flavouring agents include Raspberry Lemon flavour, Lemon flavour, Lemon/Lime flavour, Cola flavour and Orange flavour. The quantity of flavouring needed to achieve a palatable taste depends on the identity of the flavouring agent. Typically, from 0.1 to 20% by weight of flavouring is appropriate. Preferably, 1.0% to 15% by weight of flavouring is used. The exact amount of flavouring that is appropriate depends, inter alia, on the intensity of the flavouring used. For example, if Orange flavouring powder is used, an appropriate amount is from 10 to 12% by weight. If Cola flavour powder is used, an appropriate amount is from 4 to 5% by weight powder. A flavour enhancer may be used in addition to a flavouring agent. Flavour enhancers include citric acid.

A salt masking agent may also be used. Examples of salt masking agents include sodium saccharide (E954), arabic gum / acacia (E414), aspartame (E951) and neospheridin dihydrochalcone (E0959) or mixtures thereof. The quantity of salt masking agent needed to achieve a palatable taste depends on the intensity of the agent. Typically, from 1 to 6 % by weight of salt masking agent is appropriate. Preferably, from 2% to 5% by weight of salt masking agent is used, particularly 3 to 4% by weight. The exact amount of flavouring that is appropriate depends on the intensity of the salt masking agent used.

Preferred compositions according to the invention are those which comprise from 5 to 20g of PEG with a molecular weight of 2500 to 4500, 0.2 to 0.6g of sodium chloride, 0.1 to 0.3g sodium bicarbonate, 0.02 to 0.1g potassium chloride, an excipient of, for example magnesium stearate and or microcrystalline cellulose, 0.002 to 0.05g acesulfame potassium, a flavouring and a salt masker.

Alternative preferred compositions according to the invention are those which comprise from 1.0 to 5g of PEG with a molecular weight of 2500 to 4500, 0.04 to 0.15g of sodium chloride, 0.02 to 0.075g sodium bicarbonate, 0.004 to 0.025g potassium chloride, an excipient of, for example magnesium stearate and or microcrystalline cellulose, 0.0004 to 0.0125g acesulfame potassium, a flavouring and a salt masker.

The compressed pharmaceutical compositions of the invention are for oral administration. Preferably the compressed pharmaceutical compositions of the invention are in the form of tablets, pellets, pills or lozenges. In one embodiment, they may be chewable or suckable.

The invention also provides a compressed pharmaceutical composition comprising polyethylene glycol of molecular weight from 2000 to 4500 and one or more electrolytes selected from sodium chloride, sodium bicarbonate and potassium chloride, for the treatment of constipation, faecal impaction, faecal retention, intestinal gas and cramping, or flatulence; or for orthostatic lavage, colon evacuation or colon cleansing.

The compressed pharmaceutical compositions of the invention have the further advantages that they may be manufactured and packaged in a convenient manner. For example, manufacturing and packaging techniques used in the manufacture of pharmaceutical tablets may be employed.

The compressed pharmaceutical compositions of the invention are typically made by mixing together the ingredients in dry powder form and compressing into the desired shape.

The invention also provides a method for preparing a compressed pharmaceutical composition comprising the steps of:
a) mixing together polyethylene glycol of molecular weight from 2000 to 4500 with one or more electrolytes and optionally one or more ingredients selected from:,one or more sweeteners; one or more flavouring agents; one or more excipients; ascorbic acid and/or an ascorbate salt; sodium sulfate; and optional further components; and
b) compressing the mixture into a compressed pharmaceutical composition.

Methods of preparing compressed pharmaceutical compositions are well-known in the art, for example see Rudnic, E., and Schwartz, J.B. Oral Solid Dosage Forms, Chapter 92, Tablets, pp. 1615-1641, in Remington's, 19th Ed..

The invention also provides a method of treating constipation, faecal impaction, faecal retention, intestinal gas and cramping, or flatulence which comprises administering an effective dose of a compressed pharmaceutical composition according to the invention to a mammal in need of such treatment. Preferably, the mammal is a human. The invention particularly provides a method of treating constipation in a mammal.

The invention also provides a method of cleansing the colon of a mammal by administration of an effective amount of a compressed pharmaceutical composition according to the invention. Preferably, the mammal is a human.

The invention further provides the use of a compressed pharmaceutical composition according to the invention for the manufacture of a medicament for the treatment of constipation, faecal impaction, faecal retention, intestinal gas or cramping, or flatulence in a mammal. The invention also provides the use of a compressed pharmaceutical composition according to the invention for the manufacture of a medicament for cleansing the colon.

The invention further provides the use of a compressed pharmaceutical composition according to the invention for the manufacture of a medicament for the treatment of constipation.

The invention further provides the use of PEG for the manufacture of a medicament for the treatment of constipation, said medicament being a compressed pharmaceutical composition.

The following non-limiting Examples illustrate the invention.

### EXAMPLES

### A. Example formulations:

### Formulation 1

The following ingredients were mixed in powder form:

| | |
|---|---|
| Macrogol 3350 | 13.125g |
| Sodium chloride | 0.3507g |
| Sodium bicarbonate | 0.1785g |
| Potassium chloride | 0.0466g |
| Acesulfame K | 0.010g |

Formulation 1a further included Orange flavouring powder (IFF No. 17.02.1247, 11% by weight in the powder mixture, i.e. 1.508g) and salt masker (3.45% by weight in the powder mixture, i.e. 0.473g). Formulation 1b further included Cola flavour powder (IFF No. 15.02.3366, 4.4% by weight in the powder mixture, i.e. 0.603g) and salt masker (3.45% by weight in the powder mixture, i.e. 0.473g). Formulation 1c further included Raspberry Lemon flavour powder (IFF No. 10.84.8966, 1.5% by weight in the powder mixture, i.e. 0.206g, NB the Raspberry Lemon flavour powder includes salt masker).

The salt masker was the product sold by IFF under the number 13.63.0145 which comprises 6.8% sodium saccharide (E954), 4.6% arabic gum / acacia (E414), 4.1% aspartame (E951) and 0.09% neospheridin dihydrochalcone (E0959) and lactose natural and nature identical flavouring substances.

The powder mixture was then compressed.

### Formulation 2

The following ingredients were mixed in powder form:

| | |
|---|---|
| Macrogol 3350 | 13.125g |
| Sodium chloride | 0.3507g |
| Sodium bicarbonate | 0.1785g |
| Potassium chloride | 0.0466g |
| Acesulfame K | 0.010g |

Formulation 2a further included Orange flavouring powder (IFF No. 15.02.9804, 2% by weight in the powder mixture, i.e. 0.264g), salt masker (4% by weight in the powder mixture, i.e. 0.548g) and fizz powder (IFF No. 17.43.6575, 10% by weight in the powder mixture, i.e. 1.371g). Formulation 2b further included Cola flavour powder (IFF No. 15.02.3366, 1.5% by weight in the powder mixture, i.e. 0.206), salt masker (4% by weight in the powder mixture, i.e. 0.548g) and fizz powder (IFF No. 17.43.6575, 10% by weight in the powder mixture, i.e. 1.371g).

The salt masker was the product sold by IFF under the number 13.63.0145 which comprises 6.8% sodium saccharide (E954), 4.6% arabic gum / acacia (E414), 4.1% aspartame (E951) and 0.09% neospheridin dihydrochalcone (E0959) and lactose natural and nature identical flavouring substances.

The powder mixture was then compressed.

### Formulation 3

The following ingredients were mixed in powder form:

| | |
|---|---|
| Macrogol 3350 | 13.125g |
| Sodium sulphate | 0.9375g |
| Sodium chloride | 0.3507g |
| Sodium bicarbonate | 0.1785g |
| Potassium chloride | 0.0466g |
| Acesulfame K | 0.010g |

Formulation 3a further included Orange flavouring powder (IFF No. 17.02.1247, 11% by weight in the powder mixture, i.e. 1.508g) and salt masker (3.45% by weight in the powder mixture, i.e. 0.473g). Formulation 3b further included Cola flavour powder (IFF No. 15.02.3366, 4.4% by weight in the powder mixture, i.e. 0.603g) and salt masker (3.45% by weight in the powder mixture, i.e. 0.473g). Formulation 3c further included Raspberry Lemon flavour powder (IFF No. 10.84.8966, 1.5% by weight in the powder mixture, i.e. 0.206g, NB the Raspberry Lemon flavour powder includes salt masker).

The salt masker was the product sold by IFF under the number 13.63.0145 which comprises 6.8% sodium saccharide (E954), 4.6% arabic gum / acacia (E414), 4.1% aspartame (E951) and 0.09% neospheridin dihydrochalcone (E0959) and lactose natural and nature identical flavouring substances.

The powder mixture was then compressed.

### B. Taste tests

The acceptability of compositions of the invention was compared with compositions of the prior art. The prior art compositions used were Movicol® preparations, obtained from Norgine Limited (Chaplin House, Widewater Place, Moorhall Road, Harefield, Middlesex UB9 6NS, UK). It is supplied as a powder in sachets, each containing 13.8g of powder. Each sachet contains the following ingredients: Macrogol (Polyethylene Glycol) 3350: 13.125g, Sodium Chloride: 350.7mg; Sodium Bicarbonate: 178.5mg; and Potassium Chloride: 46.6mg. The contents of a sachet were made up to 125mL of solution by the addition of water.

In the taste tests, six healthy volunteers were given either one serving of Movicol preparation or one serving of formulation 1c. Each of the six volunteers said they preferred the compressed tablet to the drink solution.

## Claims

1. A compressed pharmaceutical composition comprising
- polyethylene glycol of molecular weight from 2000 to 4500, wherein the polyethylene glycol makes up from 80 to 99.5% by weight of the composition,
- one or more electrolytes
for use in the treatment of constipation, faecal impaction, faecal retention, intestinal gas and cramping, or flatulence; or for use in orthostatic lavage, colon evacuation or colon cleansing.

2. A pharmaceutical composition for the use as claimed in claim 1 wherein the electrolytes are selected from sodium chloride, sodium bicarbonate, or potassium chloride.

3. A pharmaceutical composition for the use as claimed in any one of claims 1 or 2 further comprising one or more sweeteners.

4. A pharmaceutical composition for the use as claimed in any one of claims 1 to 3 further comprising one or more flavouring agents.

5. A pharmaceutical composition for the use as claimed in any one of claims 1 to 4 which comprises from 5 to 20g of PEG with a molecular weight of 2500 to 4500, 0.1 to 1.0g of sodium chloride, 0.05 to 0.5g sodium bicarbonate, 0.01 to 0.15g potassium chloride, and optionally 0.2 to 2g of sodium sulphate.

6. A pharmaceutical composition for the use as claimed in any one of claims 1 to 4 which comprises from 1.5 to 5.5g of PEG with a molecular weight of 2500 to 4500, 0.0401 to 0.147g of sodium chloride, 0.0204 to 0.0748g sodium bicarbonate, 0.00533 to 0.0195g potassium chloride, and optionally 0.107 to 0.393g of sodium sulphate.

7. A pharmaceutical composition for the use as claimed in any one of claims 1 to 5, in unit dosage form having an amount of from 5 to 20g of polyethylene glycol, optionally in divided dose form.

8. A pharmaceutical composition for the use as claimed in any one of claims 1 to 4, in unit dosage form having an amount of from 200 to 300g of polyethylene glycol, optionally in divided dose form.

9. A method for preparing a compressed pharmaceutical composition as described in claim 1 comprising the steps of:
a) mixing together polyethylene glycol of molecular weight from 2000 to 4500 with one or more electrolytes and optionally one or more ingredients selected from: one or more sweeteners; one or more flavouring agents; one or more excipients; ascorbic acid and/or an ascorbate salt; sodium sulfate; and optional further components; and
b) compressing the mixture into a compressed pharmaceutical composition.

10. Use of a compressed pharmaceutical composition as claimed in any one of claims 1 to 6 for the manufacture of a medicament for the treatment of constipation, faecal impaction, faecal retention, intestinal gas or cramping, or flatulence.

11. Use of a compressed pharmaceutical composition as claimed in any one of claims 1 to 6 for the manufacture of a medicament for cleansing the colon.

## Patentansprüche

1. Eine gepresste pharmazeutische Zusammensetzung, umfassend
- Polyethylenglycol mit einem Molekulargewicht von 2000 bis 4500, wobei das Polyethylenglycol 80 bis 99,5 Gew.-% der Zusammensetzung ausmacht,
- einen oder mehrere Elektrolyte,
zur Verwendung bei der Behandlung von Obstipation, Koteinklemmung, Stuhlverhalt, Darmgas und -krampf oder Flatulenz, oder zur Verwendung zur orthostatischen Spülung, Kolonentleerung oder Kolonreinigung.

2. Eine pharmazeutische Zusammensetzung zur Verwendung wie in Anspruch 1 beansprucht, wobei die Elektrolyte ausgewählt sind aus Natriumchlorid, Natriumhydrogencarbonat oder Kaliumchlorid.

3. Eine pharmazeutische Zusammensetzung zur Verwendung wie in einem der Ansprüche 1 oder 2 beansprucht, ferner umfassend ein oder mehrere Süßstoffe.

4. Eine pharmazeutische Zusammensetzung zur Verwendung wie in einem der Ansprüche 1 bis 3 beansprucht, ferner umfassend ein oder mehrere Aromastoffe.

5. Eine pharmazeutische Zusammensetzung zur Verwendung wie in einem der Ansprüche 1 bis 4 beansprucht, umfassend 5 bis 20 g PEG mit einem Molekulargewicht von 2500 bis 4500, 0,1 bis 1,0 g Natriumchlorid, 0,05 bis 0,5 g Natriumhydrogencarbonat, 0,01 bis 0,15 g Kaliumchlorid und gegebenenfalls 0,2 bis 2 g Natriumsulfat.

6. Eine pharmazeutische Zusammensetzung zur Verwendung wie in einem der Ansprüche 1 bis 4 beansprucht, umfassend 1,5 bis 5,5 g PEG mit einem Molekulargewicht von 2500 bis 4500, 0,0401 bis 0,147 g Natriumchlorid, 0,0204 bis 0,0748 g Natriumhydrogencarbonat, 0,00533 bis 0,0195 g Kaliumchlorid und gegebenenfalls 0,107 bis 0,393 g Natriumsulfat.

7. Eine pharmazeutische Zusammensetzung zur Verwendung wie in einem der Ansprüche 1 bis 5 beansprucht in Einheitsdosisform mit einer Menge von 5 bis 20 g Polyethylenglycol, gegebenenfalls in Form einer geteilten Dosis.

8. Eine pharmazeutische Zusammensetzung zur Verwendung wie in einem der Ansprüche 1 bis 4 beansprucht in Einheitsdosisform mit einer Menge von 200 bis 300 g Polyethylenglycol, gegebenenfalls in Form einer geteilten Dosis.

9. Ein Verfahren zur Herstellung einer gepressten pharmazeutischen Zusammensetzung wie in Anspruch 1 beschrieben, umfassend die Schritte:
a) Vermischen von Polyethylenglycol mit einem Molekulargewicht von 2000 bis 4500 mit einem oder mehreren Elektrolyten und gegebenenfalls einem oder mehreren Bestandteilen, ausgewählt aus: einem oder mehreren Süßstoffen, einem oder mehreren Aromastoffen, einem oder mehreren Hilfsstoffen, Ascorbinsäure und/oder einem Ascorbinsäuresalz, Natriumsulfat und optionalen weiteren Komponenten, und
b) Pressen der Mischung zu einer gepressten pharmazeutischen Zusammensetzung.

10. Verwendung einer gepressten pharmazeutischen Zusammensetzung wie in einem der Ansprüche 1 bis 6 beansprucht zur Herstellung eines Medikaments zur Behandlung von Obstipation, Koteinklemmung, Stuhlverhalt, Darmgas oder -krampf oder Flatulenz.

11. Verwendung einer gepressten pharmazeutischen Zusammensetzung wie in einem der Ansprüche 1 bis 6 beansprucht zur Herstellung eines Medikaments zur Reinigung des Kolons.

## Revendications

1. Une composition pharmaceutique compressée comprenant
- du polyéthylène glycol de poids moléculaire de 2000 à 4500 dans laquelle le polyéthylène glycol forme de 80 à 99,5% en poids de la composition,
- un ou plusieurs électrolytes
destinée à un emploi pour le traitement de la constipation, de l'impaction fécale, de la rétention fécale, des gaz intestinaux et des crampes, ou de la flatulence; ou destinée à un emploi pour le lavage orthostatique, l'évacuation du colon ou le nettoyage du colon.

2. Une composition pharmaceutique pour l'emploi tel que revendiqué dans la revendication 1, dans laquelle les électrolytes sont choisis parmi le chlorure de sodium, le bicarbonate de sodium ou le chlorure de potassium.

3. Une composition pharmaceutique pour l'emploi tel que revendiqué dans l'une quelconque des revendications 1 ou 2, comprenant en outre un ou plusieurs agents édulcorants.

4. Une composition pharmaceutique pour l'emploi tel que revendiqué dans l'une quelconque des revendications 1 à 3, comprenant en outre un ou plusieurs agents aromatisants.

5. Une composition pharmaceutique pour l'emploi tel que revendiqué dans l'une quelconque des revendications 1 à 4, qui comprend de 5 à 20g de PEG avec un poids moléculaire de 2500 à 4500, 0,1 à 1,0g de chlorure de sodium, 0,05 à 0,5g de bicarbonate de sodium, 0,01 à 0,15g de chlorure de potassium et éventuellement 0,2 à 2g de sulfate de sodium.

6. Une composition pharmaceutique pour l'emploi tel que revendiqué dans l'une quelconque des revendications 1 à 4, qui comprend de 1,5 à 5,5g de PEG avec un poids moléculaire de 2500 à 4500, 0,0401 à 0,147g of chlorure de sodium, 0,0204 à 0,0748g de bicarbonate de sodium, 0,00533 à 0,0195g de chlorure de potassium, et éventuellement 0,107 à 0,393g de sulfate de sodium.

7. Une composition pharmaceutique pour l'emploi tel que revendiqué dans l'une quelconque des revendications 1 à 5, sous forme de doses unitaires ayant une quantité de 5 à 20g de polyéthylène glycol, éventuellement sous forme de doses divisées.

8. Une composition pharmaceutique pour l'emploi tel que revendiqué dans l'une quelconque des revendications 1 à 4, sous forme de doses unitaires, ayant une quantité de 200 à 300g de polyéthylène glycol, éventuellement sous forme de doses divisées.

9. Un procédé de préparation d'une composition pharmaceutique compressée telle que décrite dans la revendication 1, comprenant les étapes de:
a) mélange entre eux du polyéthylène glycol de poids moléculaire de 2000 à 4500 avec un ou plusieurs électrolytes et éventuellement un ou plusieurs ingrédients choisis parmi: un ou plusieurs agents édulcorants, un ou plusieurs agents aromatisants, un ou plusieurs excipients; de l'acide ascorbique et/ou un sel d'ascorbate; du sulfate de sodium et en outre éventuellement d'autres composés; et
b) compression du mélange en une composition pharmaceutique compressée.

10. Utilisation d'une composition pharmaceutique compressée telle que revendiquée dans l'une quelconque des revendications 1 à 6 pour la fabrication d'un médicament pour le traitement de la constipation, de l'impaction fécale, de la rétention fécale, des gaz intestinaux ou des crampes, ou de la flatulence.

11. Utilisation d'une composition pharmaceutique compressée telle que revendiquée dans l'une quelconque des revendications 1 à 6 pour la fabrication d'un médicament pour le nettoyage du colon.
